(19) Europäisches Patentamt
European Patent Office
Office européen des brevets

(11) Veröffentlichungsnummer: **0 024 017**
**A2**

# (12) EUROPÄISCHE PATENTANMELDUNG

(21) Anmeldenummer: 80104525.3

(22) Anmeldetag: 31.07.80

(51) Int. Cl.³: **C 07 D 239/26**
C 07 D 239/30, C 07 D 239/36
C 07 D 239/52, A 01 N 43/64
A 01 N 43/82

(30) Priorität: 11.08.79 DE 2932643

(43) Veröffentlichungstag der Anmeldung:
18.02.81 Patentblatt 81/7

(84) Benannte Vertragsstaaten:
BE CH DE FR GB IT LI NL

(71) Anmelder: BAYER AG
Zentralbereich Patente, Marken und Lizenzen
D-5090 Leverkusen 1, Bayerwerk(DE)

(72) Erfinder: Thomas, Rudolf, Dr.
Dellbusch 269
D-5600 Wuppertal 2(DE)

(72) Erfinder: Stetter, Jörg, Dr.
Gellertweg 4
D-5600 Wuppertal 1(DE)

(72) Erfinder: Eue, Ludwig, Dr.
Paul-Klee-Strasse 36
D-5090 Leverkusen(DE)

(72) Erfinder: Schmidt, Robert Rudolf, Dr.
Hahnenweg 5
D-5000 Köln 80(DE)

## (54) N-Pyrimidinylmethyl-halogenacetanilide, Verfahren zu ihrer Herstellung sowie ihre Verwendung als Herbizide; Zwischenprodukte und ihre Herstellung.

(57) Neue N-Pyrimidinylmethyl-halogenacetanilide der Formel

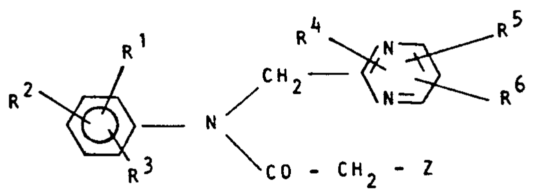

(I)

in welcher
$R^1$ für Wasserstoff oder Alkyl steht,
$R^2$ fur Wasserstoff oder Alkyl steht,
$R^3$ fur Wasserstoff oder Alkyl steht,
$R^4$ für Wasserstoff, Alkyl, Halogen, Alkoxy oder Hydroxy steht,
$R^5$ für Wasserstoff, Alkyl, Halogen, Alkoxy oder Hydroxy steht,
$R^6$ für Wasserstoff, Alkyl, Halogen, Alkoxy oder Hydroxy steht und
Z für Halogen steht,
ein Verfahren zu ihrer Herstellung sowie ihre Verwendung als Herbizide.

EP 0 024 017 A2

Croydon Printing Company Ltd

BAYER AKTIENGESELLSCHAFT 5090 Leverkusen, Bayerwerk
Zentralbereich
Patente, Marken und Lizenzen Dü/kl
Ia

BEZEICHNUNG GEÄNDERT
siehe Titelseite

N-Pyrimidinylmethyl-halogenacetanilide, Verfahren zu ihrer Herstellung sowie ihre Verwendung als Herbizide

Die vorliegende Erfindung betrifft neue N-Pyrimidinylmethyl-halogenacetanilide, ein Verfahren zu ihrer Herstellung sowie ihre Verwendung als Herbizide.

Es ist bereits bekannt geworden, daß man 2,6-Diethyl-N-methoxymethyl-chloracetanilid zur selektiven Unkrautbekämpfung verwenden kann (vgl. R. Wegler, Chemie der Pflanzenschutz- und Schädlingsbekämpfungsmittel, Band 5, Seite 255, Springer-Verlag (1977) sowie US-Patentschrift 3 442 945). Diese Verbindung wirkt hauptsächlich gegen hirseartige Gräser, z. B. Digitaria, Echinochloa etc. Andere Schadgräser, z. B. Alopecurus, werden mit obigem Wirkstoff nur in höheren Dosierungen bekämpft. Bei diesen Dosierungen treten aber an den Kulturpflanzen, z. B. an Zuckerrüben, deutliche Schäden auf, so daß der Wirkstoff in dieser Kultur nicht eingesetzt werden kann.

Es wurden neue N-Pyrimidinylmethyl-halogenacetanilide der Formel

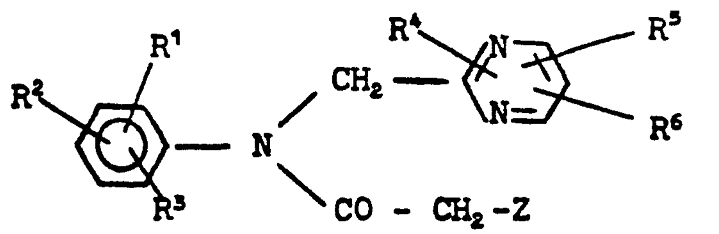

(I)

in welcher

$R^1$ für Wasserstoff oder Alkyl steht,

$R^2$ für Wasserstoff oder Alkyl steht,

$R^3$ für Wasserstoff oder Alkyl steht,

$R^4$ für Wasserstoff, Alkyl, Halogen, Alkoxy oder Hydroxy steht,

$R^5$ für Wasserstoff, Alkyl, Halogen, Alkoxy oder Hydroxy steht,

$R^6$ für Wasserstoff, Alkyl, Halogen, Alkoxy oder Hydroxy steht und

Z für Halogen steht,

gefunden.

Weiterhin wurde gefunden, daß man die N-Pyrimidinylmethyl-halogenacetanilide der Formel (I) erhält, wenn man N-Pyrimidinylmethyl-aniline der Formel

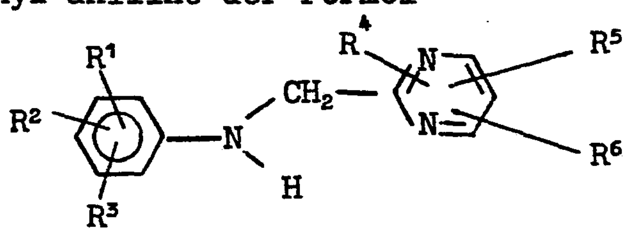

(II)

in welcher

$R^1$, $R^2$, $R^3$, $R^4$, $R^5$ und $R^6$ die oben angegebene Bedeutung haben,

mit Halogenessigsäurechloriden oder -bromiden bzw. -anhydriden der Formeln

$Z - CH_2 - CO - Cl(Br)$ (IIIa)

bzw.

$(Z - CH_2 - CO)_2O$ (IIIb)

in welchen

Z die oben angegebene Bedeutung hat,

in Gegenwart eines Verdünnungsmittels und gegebenenfalls in Gegenwart eines Säurebindemittels umsetzt.

Außerdem wurde gefunden, daß die neuen N-Pyrimidinylmethyl-halogenacetanilide der Formel (I) starke herbizide, insbesondere auch selektiv-herbizide Eigenschaften aufweisen.

Überraschenderweise zeigen die erfindungsgemäßen N-Pyrimidinylmethyl-halogenacetanilide bei sehr guter Unkrautwirkung insbesondere bessere Möglichkeiten zum Einsatz als selektive Unkrautbekämpfungsmittel in wichtigen Kulturpflanzen als das aus dem Stand der Technik bekannte 2,6-Diethyl-N-methoxymethyl-chloracetanilid, welches ein hochaktiver Wirkstoff gleicher Wirkungsart ist. So sind die erfindungsgemäßen Wirkstoffe gegen Gräser wie Alopecurus und Poa besser wirksam als die genannte Vergleichskomponente. Außerdem sind sie für Rüben deutlich verträglicher als das 2,6-Diethyl-N-methoxy=methyl-chloracetanilid. Mit den erfindungsgemäßen Stoffen ist es daher, - im Gegensatz zu dem bekannten 2,6-Diethyl-N-methoxymethyl-chloracetanilid -, möglich, Gräser wie Alopecurus, Poa und hirseartige Gräser wie Digitaria, Echinochloa und Setaria gleichzeitig in Rüben zu bekämpfen, aber auch in Baumwolle und Sojabohnen. Die erfindungsgemäßen Stoffe stellen somit eine wertvolle Bereicherung der Technik dar.

Die erfindungsgemäßen N-Pyrimidinylmethyl-halogenacet=anilide sind durch die Formel (I) allgemein definiert. In dieser Formel sind $\underline{R}^1$, $\underline{R}^2$ und $\underline{R}^3$ gleich oder verschieden und stehen vorzugsweise für Wasserstoff und geradkettiges oder verzweigtes Alkyl mit 1 bis 4 Kohlenstoffatomen. $\underline{R}^4$, $\underline{R}^5$ und $\underline{R}^6$ sind gleich oder verschieden und stehen vorzugsweise für Wasserstoff, geradkettiges oder verzweigtes Alkyl und Alkoxy mit jeweils 1 bis 4 Kohlenstoffatomen, Chlor oder Hydroxy.
$\underline{Z}$ steht vorzugsweise für Chlor, Brom und Jod.

Ganz besonders bevorzugt sind diejenigen N-Pyrimidinyl=methyl-halogenacetanilide der Formel (I), in denen $\underline{R}^1$ für Wasserstoff, Methyl, Ethyl, n-Propyl, Isopropyl, sek.-Butyl oder tert.-Butyl steht; $\underline{R}^2$ für Wasserstoff, Methyl, Ethyl, n-Propyl, Isopropyl, sek.-Butyl oder tert.-Butyl steht; $\underline{R}^3$ für Wasserstoff, Methyl, Ethyl,

n-Propyl, Isopropyl, sek.-Butyl oder tert.-Butyl steht; $\underline{R}^4$ für Wasserstoff, Methyl, Ethyl, Isopropyl, Methoxy, Chlor oder Hydroxy steht; $\underline{R}^5$ für Wasserstoff, Methyl, Ethyl, Isopropyl, Methoxy, Chlor oder Hydroxy steht; $\underline{R}^6$ für Wasserstoff, Methyl, Ethyl, Isopropyl, Methoxy, Chlor oder Hydroxy steht; und $\underline{Z}$ für Chlor oder Brom steht.

Im einzelnen seien außer den bei den Herstellungsbeispielen genannten Verbindungen die folgenden Verbindungen der allgemeinen Formel (I) genannt:

Tabelle 1

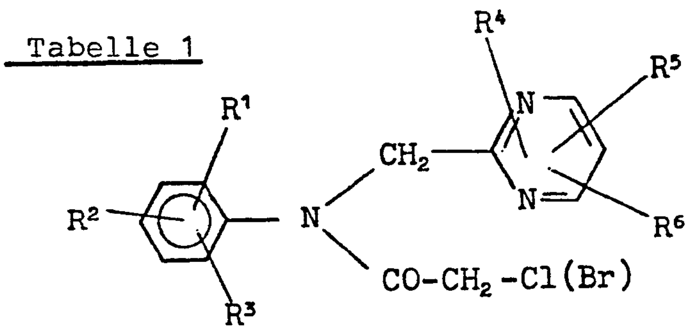

(Ia)

| $R^1$ | $R^2$ | $R^3$ | $R^4$ | $R^5$ | $R^6$ |
|---|---|---|---|---|---|
| 2-$C_2H_5$ | 6-$C_2H_5$ | H | H | H | H |
| 2-$CH_3$ | H | H | H | H | H |
| 2-$C(CH_3)_3$ | H | H | H | H | H |
| 2-$CH_3$ | 3-$CH_3$ | H | H | H | H |
| 2-$CH_3$ | 5-$CH_3$ | H | H | H | H |
| 2-$CH(CH_3)_2$ | H | H | H | H | H |
| 2-$CH_3$ | 6-$CH_3$ | H | 4-$CH_3$ | H | H |
| 2-$CH_3$ | 6-$C_2H_5$ | H | 4-$CH_3$ | H | H |
| 2-$C_2H_5$ | 6-$C_2H_5$ | H | 4-$CH_3$ | H | H |
| 2-$CH_3$ | H | H | 4-$CH_3$ | H | H |

0024017

Tabelle 1 (Fortsetzung)

| $R^1$ | $R^2$ | $R^3$ | $R^4$ | $R^5$ | $R^6$ |
|---|---|---|---|---|---|
| 2-$C(CH_3)_3$ | H | H | 4-$CH_3$ | H | H |
| 2-$CH_3$ | 3-$CH_3$ | H | 4-$CH_3$ | H | H |
| 2-$CH_3$ | 5-$CH_3$ | H | 4-$CH_3$ | H | H |
| 2-$CH(CH_3)_2$ | H | H | 4-$CH_3$ | H | H |
| 2-$CH_3$ | 6-$CH_3$ | H | 4-$CH_3$ | 6-$CH_3$ | H |
| 2-$CH_3$ | 6-$C_2H_5$ | H | 4-$CH_3$ | 6-$CH_3$ | H |
| 2-$C_2H_5$ | 6-$C_2H_5$ | H | 4-$CH_3$ | 6-$CH_3$ | H |
| 2-$CH_3$ | H | H | 4-$CH_3$ | 6-$CH_3$ | H |
| 2-$C(CH_3)_3$ | H | H | 4-$CH_3$ | 6-$CH_3$ | H |
| 2-$CH_3$ | 3-$CH_3$ | H | 4-$CH_3$ | 6-$CH_3$ | H |
| 2-$CH_3$ | 5-$CH_3$ | H | 4-$CH_3$ | 6-$CH_3$ | H |
| 2-$CH(CH_3)_2$ | H | H | 4-$CH_3$ | 6-$CH_3$ | H |
| 2-$CH_3$ | 6-$CH_3$ | H | 4-OH | 6-$CH_3$ | H |
| 2-$CH_3$ | 6-$C_2H_5$ | H | 4-OH | 6-$CH_3$ | H |
| 2-$C_2H_5$ | 6-$C_2H_5$ | H | 4-OH | 6-$CH_3$ | H |
| 2-$CH_3$ | H | H | 4-OH | 6-$CH_3$ | H |
| 2-$C(CH_3)_3$ | H | H | 4-OH | 6-$CH_3$ | H |
| 2-$CH_3$ | 3-$CH_3$ | H | 4-OH | 6-$CH_3$ | H |
| 2-$CH_3$ | 5-$CH_3$ | H | 4-OH | 6-$CH_3$ | H |
| 2-$CH(CH_3)_2$ | H | H | 4-OH | 6-$CH_3$ | H |
| 2-$CH_3$ | 6-$CH_3$ | H | 4-OH | 5-$OCH_3$ | H |
| 2-$CH_3$ | 6-$C_2H_5$ | H | 4-OH | 5-$OCH_3$ | H |
| 2-$C_2H_5$ | 6-$C_2H_5$ | H | 4-OH | 5-$OCH_3$ | H |
| 2-$CH_3$ | H | H | 4-OH | 5-$OCH_3$ | H |
| 2-$C(CH_3)_3$ | H | H | 4-OH | 5-$OCH_3$ | H |
| 2-$CH_3$ | 3-$CH_3$ | H | 4-OH | 5-$OCH_3$ | H |
| 2-$CH_3$ | 5-$CH_3$ | H | 4-OH | 5-$OCH_3$ | H |
| 2-$CH(CH_3)_2$ | H | H | 4-OH | 5-$OCH_3$ | H |

Tabelle 1 (Fortsetzung)

| $R^1$ | $R^2$ | $R^3$ | $R^4$ | $R^5$ | $R^6$ |
|---|---|---|---|---|---|
| 2-$CH_3$ | 6-$CH_3$ | H | 4-OH | 5-$CH_3$ | 6-$CH_3$ |
| 2-$CH_3$ | 6-$C_2H_5$ | H | 4-OH | 5-$CH_3$ | 6-$CH_3$ |
| 2-$C_2H_5$ | 2-$C_2H_5$ | H | 4-OH | 5-$CH_3$ | 6-$CH_3$ |
| 2-$CH_3$ | H | H | 4-OH | 5-$CH_3$ | 6-$CH_3$ |
| 2-$C(CH_3)_3$ | H | H | 4-OH | 5-$CH_3$ | 6-$CH_3$ |
| 2-$CH_3$ | 3-$CH_3$ | H | 4-OH | 5-$CH_3$ | 6-$CH_3$ |
| 2-$CH_3$ | 5-$CH_3$ | H | 4-OH | 5-$CH_3$ | 6-$CH_3$ |
| 2-$CH(CH_3)_3$ | H | H | 4-OH | 5-$CH_3$ | 6-$CH_3$ |
| 2-$CH_3$ | 6-$CH_3$ | H | 4-$CH_3$ | 5-Cl | 6-OH |
| 2-$CH_3$ | 6-$C_2H_5$ | H | 4-$CH_3$ | 5-Cl | 6-OH |
| 2-$C_2H_5$ | 6-$C_2H_5$ | H | 4-$CH_3$ | 5-Cl | 6-OH |
| 2-$CH_3$ | H | H | 4-$CH_3$ | 5-Cl | 6-OH |
| 2-$C(CH_3)_3$ | H | H | 4-$CH_3$ | 5-Cl | 6-OH |
| 2-$CH_3$ | 3-$CH_3$ | H | 4-$CH_3$ | 5-Cl | 6-OH |
| 2-$CH_3$ | 5-$CH_3$ | H | 4-$CH_3$ | 5-Cl | 6-OH |
| 2-$CH(CH_3)_2$ | H | H | 4-$CH_3$ | 5-Cl | 6-OH |

Verwendet man beispielsweise N-(Pyrimidin-2-yl-methyl)-2,6-dimethyl-anilin und Chloracetylchlorid als Ausgangsstoffe, so kann der Reaktionsablauf durch das folgende Formelschema wiedergegeben werden:

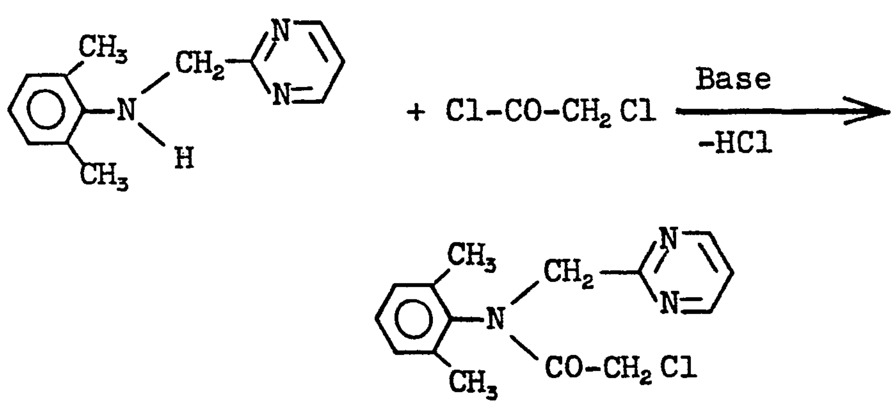

Die bei der Durchführung des erfindungsgemäßen Verfahrens als Ausgangsstoffe benötigten N-Pyrimidinylmethyl-aniline sind durch die Formel (II) allgemein definiert. In dieser Formel stehen $\underline{R}^1$, $\underline{R}^2$, $\underline{R}^3$, $\underline{R}^4$, $\underline{R}^5$ und $\underline{R}^6$ vorzugsweise für diejenigen Reste, die bereits im Zusammenhang mit der Beschreibung der erfindungsgemäßen Stoffe der Formel (I) vorzugsweise für diese Reste genannt wurden.

Die N-Pyrimidinylmethyl-aniline der Formel (II) sind noch nicht bekannt. Man erhält sie, wenn man Aniline der Formel

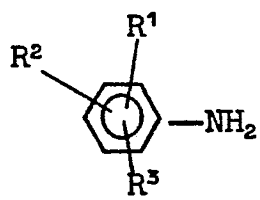

(IV)

in welcher

$R^1$, $R^2$ und $R^3$ die oben angegebene Bedeutung haben,

mit Pyrimidin-2-yl-methyl-chloriden der Formel

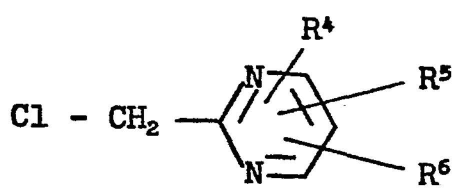

(V)

in welcher

$R^4$, $R^5$ und $R^6$ die oben angegebene Bedeutung haben,

in Gegenwart eines Säurebinders und gegebenenfalls in Gegenwart eines Verdünnungsmittels umsetzt.

Die bei der Herstellung der N-Pyrimidinylmethyl-aniline der Formel (II) als Ausgangsstoffe benötigten Aniline der Formel (IV) sind allgemein bekannte Verbindungen der organischen Chemie. Als Beispiele seien genannt:

Tabelle 2

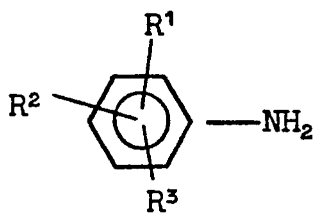

(IV)

| $R^1$ | $R^2$ | $R^3$ | $R^1$ | $R^2$ | $R^3$ |
|---|---|---|---|---|---|
| H | H | H | 2-$CH_3$ | 6-$C_4H_9$-sek. | H |
| 2-$CH_3$ | H | H | 2-$CH_3$ | 6-$C_4H_9$-sek. | H |
| 2-$C_2H_5$ | H | H | 3-$CH_3$ | 5-$CH_3$ | H |
| 2-$CH_3$ | 6-$CH_3$ | H | 2-$CH_3$ | 4-$C_2H_5$ | 6-$CH_3$ |
| 2-$CH_3$ | 6-$C_2H_5$ | H | 2-$C_2H_5$ | 4-$CH_3$ | 6-$C_2H_5$ |
| 2-$C_2H_5$ | 6-$C_2H_5$ | H | 2-$C_2H_5$ | 3-$CH_3$ | 6-$C_2H_5$ |
| 2-$CH_3$ | 3-$CH_3$ | H | 2-$C_2H_5$ | 4-$CH_3$ | 6-$CH_3$ |
| 2-$C_2H_5$ | 3-$CH_3$ | H | 2-$C_3H_7$ i | H | H |
| 2-$CH_3$ | 6-$C_3H_7$ i | H | 2-$C_3H_7$ i | 6-$C_3H_7$ i | H |
| 2-$C_2H_5$ | 4-$CH_3$ | H | 2-$C(CH_3)_3$ | 4-$CH_3$ | H |
| 2-$C_3H_7$ i | 3-$CH_3$ | H | 2-$C_3H_7$ n | H | H |
| 2-$C_3H_7$ i | 4-$CH_3$ | H | 2-$C_4H_9$-sek. | H | H |

Tabelle 2 (Fortsetzung)

| $R^1$ | $R^2$ | $R^3$ | $R^1$ | $R^2$ | $R^3$ |
|---|---|---|---|---|---|
| 2-$C_3H_7$i | 4-$CH_3$ | 6-$C_3H_7$i | 2-$C_4H_9$-sek. | 6-$C_4H_5$-sek. | H |
| 2-$C_3H_7$i | 6-$C_2H_5$ | H | 2-$C_4H_9$-sek. | 6-$C_2H_5$ | H |
| 2-$C_3H_7$i | 6-$CH_3$ | H | 2-$C(CH_3)_3$ | H | H |

Die außerdem bei der Herstellung der N-Pyrimidinylmethyl-aniline der Formel (II) als Ausgangsstoffe benötigten Pyrimidin-2-yl-methyl-chloride der Formel (V) sind ebenfalls bekannt (vergleiche z.B. US-Patentschrift 3 118 889; J.Amer.Chem.Soc. 68, 2393 (1946); Z.obsc.Chim. 34, 2164 (1964); Z.obsc.Chim. 32, 2431 (1962); Z.obsc.Chim.33, 2848 (1963); J.Med.Chem. 7, 808 (1964)) bzw. können sie nach den dort angegebenen Verfahren hergestellt werden. So wird das unsubstituierte Pyrimidin-2-yl-methyl-chlorid erhalten, wenn man Chloracetamidin-hydrochlorid (vgl.J.Org.Chem. 26, 412 (1967) mit Tetramethoxypropan umsetzt. Die Alkyl-substituierten Pyrimidin-2-yl-methyl-chloride der Formel (II) können erhalten werden, indem man das bekannte Chloracetamidin-hydrochlorid mit entsprechenden Acylketonen, Acylaldehyden bzw. deren Acetalen, wie z.B. Acetylaceton oder 3-Ketobutyraldehyd-dimethylacetal, umsetzt. Die Hydroxy-substituierten Pyrimidin-2-yl-methyl-chloride der Formel (II) können durch Umsetzung des Chloracetamidin-hydrochlorids mit Ketocarbonsäureestern, wie z.B. Acetessigester oder Malonester, erhalten werden, wobei die Hydroxy-Gruppen in üblicher Weise gegen Halogen, insbesondere Chlor, ausgetauscht werden können, wie beispeilsweise durch weitere Umsetzung mit Phosphoroxychlorid oder Thionylchlorid.

Die Halogen-substituierten Pyrimidin-2-yl-methyl-chloride der Formel (II) können auch direkt erhalten werden, indem man die gegebenenfalls Alkyl- und/oder Hydroxy-substituierten Pyrimidin-2-yl-methyl-chloride der Formel (II) in üblicher Weise halogeniert, wie z.B. durch Umsetzung mit Chlorlauge, Chlor in Tetrachlorkohlenstoff oder Sulfurylchlorid.
Die Alkoxy-substituierten Pyrimidin-2-yl-methyl-chloride der Formel (II) können z.B. erhalten werden, indem man Chloracetamindin-hydrochlorid mit α-Alkoxy-α-formylessigsäureestern, wie beispielsweise α-Methoxy-α-formylessigsäure-methylester, in üblicher Weise umsetzt.

Im einzelnen seien außer den bei den Herstellungsbeispielen genannten Verbindungen die folgenden Verbindungen der allgemeinen Formel (V) genannt:

Tabelle 3

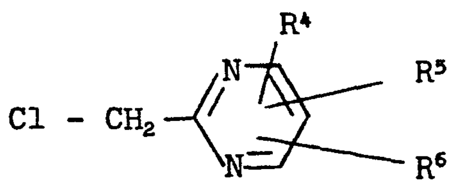

(V)

| $R^4$ | $R^5$ | $R^6$ |
|---|---|---|
| 4-$CH_3$ | 6-Cl | H |
| 4-OH | 6-OH | H |
| 4-Cl | 6-Cl | H |
| 4-Cl | H | H |

Bei der Herstellung der N-Pyrimidinylmethyl-aniline der Formel (II) nach dem oben angegebenen Verfahren, können als Säurebinder alle üblichen organischen und anorganischen Säureakzeptoren verwendet werden. Vorzugsweise in Betracht kommen: Alkalicarbonate, wie Kalium- oder Natriumcarbonat; tertiäre Amine, wie Triethylamin; oder Pyridin.

Als Verdünnungsmittel können bei der Herstellung der N-Pyrimidinylmethyl-aniline der Formel (II) alle üblichen inerten organischen Lösungsmittel eingesetzt werden. Vorzugsweise in Betracht kommen Dimethylformamid und Toluol.

Die Reaktionstemperaturen können bei der Herstellung der N-Pyrimidinylmethyl-aniline der Formel (II) nach dem obigen Verfahren innerhalb eines größeren Bereiches variiert werden. Im allgemeinen arbeitet man zwischen 0°C und 180°C, vorzugsweise zwischen 20°C und 150°C.

Bei der Herstellung der N-Pyrimidinylmethyl-aniline der Formel (II) nach dem obigen Verfahren setzt man die Aniline der Formel (IV) und die Pyrimidin-2-yl-methylchloride der Formel (V) im allgemeinen in äquimolaren Mengen ein. Es ist jedoch auch möglich, eine der Komponenten, vorzugsweise das Anilin der Formel (IV), in einem Ueberschuß einzusetzen.
Die Aufarbeitung und Isolierung der Reaktionsprodukte erfolgt nach üblichen Methoden (vergleiche auch die Herstellungsbeispiele).

Die außerdem für das erfindungsgemäße Verfahren als Ausgangsstoffe zu verwendenden Halogenessigsäurechloride und -bromide bzw. -anhydride sind durch die Formeln (IIIa) und (IIIb) allgemein definiert. In diesen Formeln steht Z vorzugsweise für Chlor, Brom und Jod.

Die Halogenessigsäurechloride und -bromide bzw. -anhydride der Formeln (IIIa) und (IIIb) sind allgemein bekannte Verbindungen der organischen Chemie. Als Beispiele seien genannt: Chloracetylchlorid, Bromacetylchlorid, Jodacetylchlorid und die entsprechenden Bromide sowie Anhydride.

Als Verdünnungsmittel kommen für die erfindungsgemäße Umsetzung vorzugsweise inerte organische Lösungsmittel infrage. Hierzu gehören vorzugsweise Ketone, wie Diethylketon, insbesondere Aceton und Methylethylketon; Nitrile, wie Propionitril, insbesondere Acetonitril; Ether, wie Tetrahydrofuran oder Dioxan; aliphatische und aromatische Kohlenwasserstoffe, wie Petrolether, Benzol, Toluol oder Xylol; halogenierte Kohlenwasserstoffe, wie Methylenchlorid, Tetrachlorkohlenstoff, Chloroform oder Chlorbenzol; und Ester, wie Essigester.

Das erfindungsgemäße Verfahren kann gegebenenfalls in Gegenwart von Säurebindern durchgeführt werden. Als solche können alle üblichen Säurebindemittel verwendet werden. Hierzu gehören vorzugsweise organische Basen, wie tertiäre Amine, beispielsweise Triethylamin, oder Pyridin, ferner anorganische Basen, wie beispielsweise Alkalihydroxide und Alkalicarbonate.

Die Reaktionstemperaturen können bei der Durchführung des erfindungsgemäßen Verfahrens in einem größeren Bereich variiert werden. Im allgemeinen arbeitet man zwischen 0°C und 120°C, vorzugsweise zwischen 20°C und 100°C.

Bei der Durchführung des erfindungsgemäßen Verfahrens setzt man vorzugsweise auf 1 Mol der Verbindung der Formel (II) 1 bis 1,5 Mol Halogenierungsmittel und 1 bis 1,5 Mol Säurebinder ein. Die Isolierung der Verbindungen der Formel (I) erfolgt in üblicher Weise.

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:

Dikotyle Unkräuter der Gattungen: Sinapsis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cuburbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-, Citrus-, Nuss-, Bananen-, Kaffee-, Tee-, Gummi-, Oelpalm-, Kakao-, Beerenfrucht- und Hopfenanlagen und zur selektiven Unkrautbekämpfung in einjährigen Kulturen eingesetzt werden.

Besonders geeignet sind die erfindungsgemäßen Wirkstoffe zum selektiven Einsatz in Beta-Rüben, Raps und anderen Brassicaceae, Sojabohnen und anderen Leguminosen sowie in Mais und Baumwolle.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsionskonzentrate, Wirkstoff-imprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln, und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzol, Chlorethylen oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, starke polare Lösungsmittel, wie Dimethylformamid und Dimethylsulfoxid, sowie Wasser.
Als feste Trägerstoffe kommen in Frage: z.B. natürliche Gesteinsmehle, wie Kaoline, Tonerden, Talkum, Kreide, Quarz, Attapilgit, Montmorillonit oder Diatomeenerde und synthetische Gesteinsmehle, wie hochdisperse Kieselsäure,

Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Die Anwendung wird vorzugsweise vor dem Auflaufen der Pflanzen, also im pre-emergence-Verfahren, vorgenommen. Sie können auch vor der Saat in den Boden eingearbeitet werden.

Die aufgewandte Wirkstoffmenge kann in größeren Bereichen schwanken. Sie hängt im wesentlichen von der Art des gewünschten Effektes ab. Im allgemeinen liegen die Aufwandmengen zwischen 0,1 und 10 kg Wirkstoff pro ha, vorzugsweise zwischen 0,25 und 5 kg/ha.

Wirkung und Einsatzmöglichkeiten zur Unkrautbekämpfung gehen aus dem Beispiel A hervor.

In dem nachfolgenden Beispiel wird die nachstehend angegebene Verbindung als Vergleichssubstanz eingesetzt:

A =

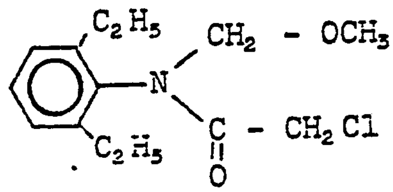

(2,6-Diethyl)-N-methoxymethyl-chloracetanilid

Beispiel A

Pre-emergence-Test

Lösungsmittel: 5 Gewichtsteile Aceton

Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 %=keine Wirkung (wie unbehandelte Kontrolle)

100 %=totale Vernichtung

Der erfindungsgemäße Wirkstoff (5) zeigt in diesem Test eine bessere selektiv herbizide Wirksamkeit als die aus dem Stand der Technik bekannte Substanz (A).

## Herstellungsbeispiele

### Beispiel 1

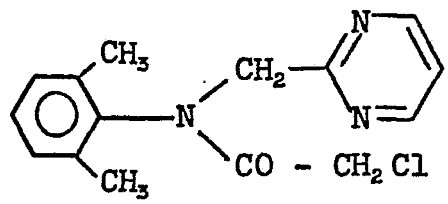

8,5g (0,04 Mol) N-(Pyrimidin-2-yl-methyl)-2,6-dimethylanilin und 3,2g (0,04 Mol) Pyridin werden in 20 ml Tetrahydrofuran unter Rückfluß erhitzt. Dazu werden 4,5g (0,04 Mol) Chloracetylchlorid getropft. Man läßt die Reaktionsmischung 5 Stunden unter Rückfluß rühren und gießt sie anschließend auf 300g Eis. Der entstehende kristalline Niederschlag wird abfiltriert, mit Wasser gewaschen und aus Tetrachlorkohlenstoff umkristallisiert. Man erhält 8,2g (71 % der Theorie) 2,6-Dimethyl-N-(pyrimidin-2-yl-methyl)-chloracetanilid in Form farbloser Kristalle vom Schmelzpunkt 110°C.

### Herstellung der Vorstufen

(II-1)

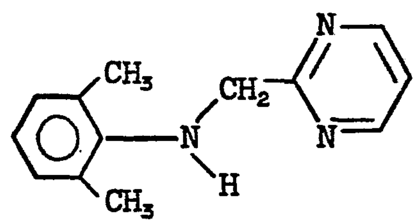

48,4g (0,4 Mol) 2,6-Dimethylanilin, 55,3g (0,4 Mol) Kaliumcarbonat und 0,2g Kaliumjodid werden in 60 ml Dimethylformamid auf 100°C erhitzt und dann tropfenweise mit 25,7g (0,2 Mol) 2-Chlormethyl-pyrimidin versetzt. Man läßt 5 Stunden bei 100°C nachrühren und gießt die Reaktionsmischung danach auf 500g Eis. Anschließend wird dreimal mit je 150ml

Methylenchlorid extrahiert. Die vereinigten organischen Phasen werden über Natriumsulfat getrocknet und im Vakuum eingeengt. Der Rückstand wird im Hochvakuum destilliert. Man erhält 30g (70 % der Theorie) N-(Pyrimidin-2-yl-methyl)-2,6-dimethylanilin als farbloses Oel vom Siedepunkt 150°C/0,2 Torr.

(V-1) 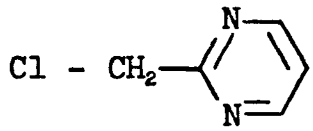

141,9 g (1,1 Mol) Chloracetamidin-hydrochlorid und 69g (0,5 Mol) Kaliumcarbonat werden in 200 ml Toluol auf 80°C erhitzt. Dann werden 164g (1 Mol) 1,3-Tetramethoxypropan zugetropft. Man destilliert dabei das entstehende Methanol ab und destilliert anschließend unter Steigerung der Badtemperatur und Erhitzen des aus dem Reaktionsgefäß mitgerissenen Toluols bis die Siedetemperatur von reinem Toluol erreicht wird. Man dekantiert das Toluol und erhält eine schwarze kristalline Masse, die noch zweimal mit je 200 ml Toluol ausgekocht wird. Die Toluolextrakte werden im Vakuum eingedampft und der dabei erhaltene Rückstand wird im Hochvakkum destilliert. Man erhält 45g (35 % der Theorie) 2-Chlormethyl-pyrimidin als farbloses Oel vom Siedepunkt 34°C/0,2 Torr.

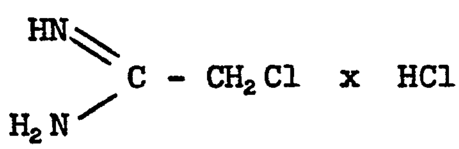

4,6g (0,2 Mol) Natrium werden in 500 ml Methanol gelöst. Unter Kühlung auf ca. 20°C tropft man 151g (2 Mol) Chloracetonitril zu und rührt anschließend 1 Stunde bei Raumtemperatur nach. Dann gibt man portionsweise 117,7g (2,2 Mol) Ammoniumchlorid zu und rührt wiederum eine Stunde

nach. Anschließend wird die Lösung im Vakuum eingedampft. Der Rückstand wird mit Ether aufgerührt, abgesaugt und mit Ether nachgewaschen.
Man erhält 255g (99 % der Theorie) Chloracetamidin-hydrochlorid in Form dunkler Kristalle vom Schmelzpunkt 98°C.

In entsprechender Weise werden diejenigen Verbindungen erhalten, die in der Tabelle 4 formelmäßig aufgeführt sind.

Tabelle 4

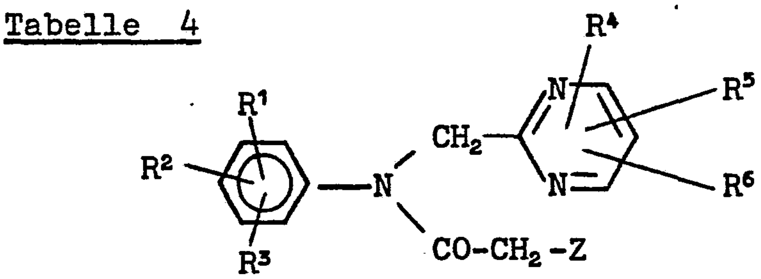

(I)

| Bsp. Nr. | $R^1$ | $R^2$ | $R^3$ | $R^4$ | $R^5$ | $R^6$ | Z | Schmelzpunkt |
|---|---|---|---|---|---|---|---|---|
| 2 | H | H | H | H | H | H | Cl | 67 |
| 3 | H | H | H | 4-$CH_3$ | H | H | Cl | 97 |
| 4 | 2-$CH_3$ | 6-$CH_3$ | H | 4-$CH_3$ | 6-$CH_3$ | H | Cl | 105 |
| 5 | 2-$CH_3$ | 6-$C_2H_5$ | H | H | H | H | Cl | 68 |

Nach dem in der Anmeldung beschriebenen Verfahren und entsprechend Beispiel 1 werden die in der nachfolgenden Tabelle 5 formelmäßig aufgeführten Ausgangsprodukte der Formel (II) erhalten:

Tabelle 5

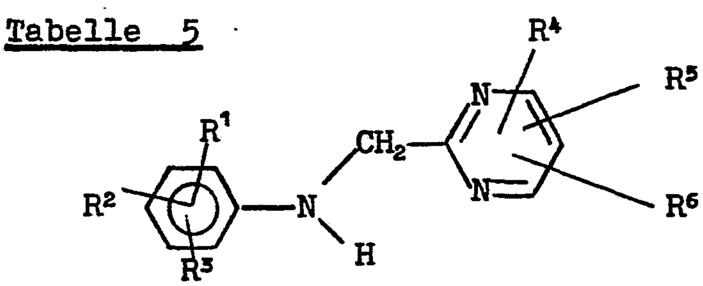

(II)

| Bsp. Nr. | $R^1$ | $R^2$ | $R^3$ | $R^4$ | $R^5$ | $R^6$ | Physikal. Konstante |
|---|---|---|---|---|---|---|---|
| (II-2) | H | H | H | H | H | H | Fp:58°C |
| (II-3) | H | H | H | 4-$CH_3$ | H | H | Fp:48°C |
| (II-4) | 2-$CH_3$ | 6-$CH_3$ | H | 4-$CH_3$ | 6-$CH_3$ | H | kp:147°C/0,15Torr |
| (II-5) | 2-$CH_3$ | 6-$C_2H_5$ | H | H | H | H | Kp:145-47°C/ 0,4 Torr |
| (II-6) | 2-$CH_3$ | 6-$CH_3$ | H | H | H | H | Kp:150°C/ 0,2Torr |

Nach den in der Anmeldung beschriebenen Verfahren werden die in der nachfolgenden Tabelle 6 formelmäßig aufgeführten Ausgangsprodukte der Formel (V) erhalten:

Tabelle 6:

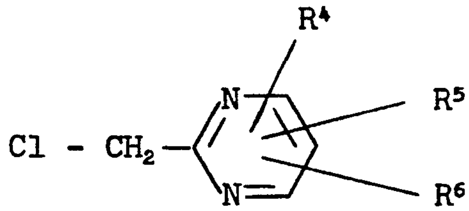

(V)

| Bsp. Nr. | $R^4$ | $R^5$ | $R^6$ | Physikal. Konstante |
|---|---|---|---|---|
| (V-2) | 4-$CH_3$ | 6-$CH_3$ | H | Fp:60°C |
| (V-3) | 4-$CH_3$ | H | H | Kp:40°C/0,1Torr |
| (V-4) | 4-$CH_3$ | 6-OH | H | Fp:160°C |
| (V-5) | 4-OH | 5-Cl | 6-$CH_3$ | Fp:179°C |
| (V-6) | 4-OH | 5-$CH_3$ | 6-$CH_3$ | Fp:267°C |
| (V-7) | 4-Cl | 5-Cl | 6-Cl | Kp:120°C/6 Torr |
| (V-8) | 4-Cl | 5-$CH_3$ | 6-Cl | Fp:40°C |
| (V-9) | 4-OH | 5-$OCH_3$ | H | Fp:138°C |

Patentansprüche

1. N-Pyrimidinylmethyl-halogenacetanilide der Formel

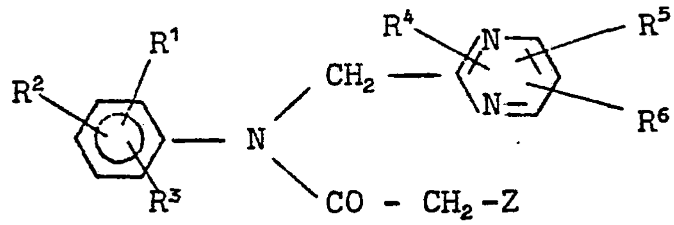

(I)

in welcher

$R^1$ für Wasserstoff oder Alkyl steht,

$R^2$ für Wasserstoff oder Alkyl steht,

$R^3$ für Wasserstoff oder Alkyl steht,

$R^4$ für Wasserstoff, Alkyl, Halogen, Alkoxy oder Hydroxy steht,

$R^5$ für Wasserstoff, Alkyl, Halogen, Alkoxy oder Hydroxy steht,

$R^6$ für Wasserstoff, Alkyl, Halogen, Alkoxy oder Hydroxy steht und

Z für Halogen steht.

2. N-Pyrimidinylmethyl-halogenacetanilide der Formel (I) gemäß Anspruch 1, in welcher $R^1$ für Wasserstoff oder Alkyl mit 1 bis 4 Kohlenstoffatomen steht, $R^2$ für Wasserstoff oder Alkyl mit 1 bis 4 Kohlenstoffatomen steht, $R^3$ für Wasserstoff oder Alkyl mit 1 bis 4 Kohlenstoffatomen steht, $R^4$ für Wasserstoff, Alkyl mit 1 bis 4 Kohlenstoffatomen, Alkoxy mit 1 bis 4 Kohlenstoffatomen, Chlor oder Hydroxy steht, $R^5$ für Wasserstoff, Alkyl mit 1 bis 4 Kohlenstoffatomen, Alkoxy mit 1 bis 4 Kohlenstoffatomen, Chlor oder Hydroxy steht, $R^6$ für Wasserstoff, Alkyl mit 1 bis 4 Kohlenstoffatomen, Alkoxy mit 1 bis 4 Kohlenstoffatomen, Chlor oder Hydroxy steht und Z für Chlor, Brom oder Jod steht.

3. N-Pyrimidinylmethyl-halogenacetanilide der Formel (I) gemäß Anspruch 1, in welcher $R^1$ für Wasserstoff, Methyl, Ethyl, n-Propyl, Isopropyl, sek.-Butyl oder tert.-Butyl, $R^2$ für Wasserstoff, Methyl, Ethyl, n-Propyl, Isopropyl, sek.-Butyl oder tert.-Butyl steht, $R^3$ für Wasserstoff, Methyl, Ethyl, n-Propyl, Isopropyl, sek.-Butyl oder tert.-Butyl steht, $R^4$ für Wasserstoff, Methyl, Ethyl, Isopropyl, Methoxy, Chlor oder Hydroxy steht, $R^5$ für Wasserstoff, Methyl, Ethyl, Isopropyl, Methoxy, Chlor oder Hydroxy steht, $R^6$ für Wasserstoff, Methyl, Ethyl, Isopropyl, Methoxy, Chlor oder Hydroxy steht und Z für Chlor oder Brom steht.

4. Verfahren zur Herstellung von N-Pyrimidinylmethyl-halogenacetaniliden der Formel (I), dadurch gekennzeichnet, daß man N-Pyrimidinylmethyl-aniline der Formel

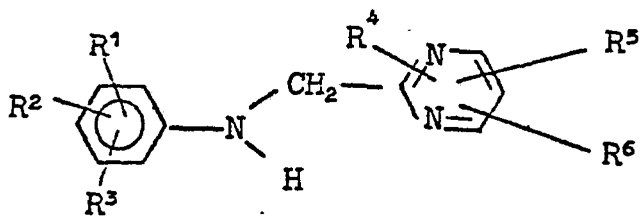

(II)

in welcher

$R^1$, $R^2$, $R^3$, $R^4$, $R^5$ und $R^6$ die oben angegebene Bedeutung haben,

mit Halogenessigsäurechloriden und -bromiden bzw. -anhydriden der Formeln

$$Z - CH_2 - CO - Cl(Br) \qquad (IIIa)$$

bzw.

$$(H - CH_2 - CO)_2O \qquad (IIIb)$$

in welchen

Z die oben angegebene Bedeutung hat,

in Gegenwart eines Verdünnungsmittels und gegebenenfalls in Gegenwart eines Säurebindemittels umsetzt.

5. Herbizide Mittel, gekennzeichnet durch einen Gehalt an mindestens einem N-Pyrimidinylmethyl-halogenacetanilid der Formel (I) gemäß Anspruch 1.

6. Verfahren zur Bekämpfung von Unkräutern, dadurch gekennzeichnet, daß man N-Pyrimidinylmethyl-halogenacetanilide der Formel (I) gemäß Anspruch 1 auf die Unkräuter und/oder deren Lebensraum ausbringt.

7. Verwendung von N-Pyrimidinylmethyl-halogenacetaniliden der Formel (I) gemäß Anspruch 1 zur Bekämpfung von Unkräutern.

8. Verfahren zur Herstellung herbzider Mittel, dadurch gekennzeichnet, daß man N-Pyrimidinylmethyl-halogenacetanilide der Formel (I) gemäß Anspruch 1 mit Streckmitteln und/oder oberflächenaktiven Stoffen vermischt.

9. N-Pyrimidinylmethyl-aniline der Formel

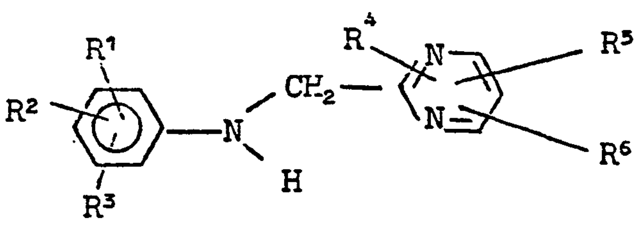

(II)

in welcher

$R^1$ für Wasserstoff oder Alkyl steht,

$R^2$ für Wasserstoff oder Alkyl steht,

$R^3$ für Wasserstoff oder Alkyl steht,

$R^4$ für Wasserstoff, Alkyl, Halogen, Alkoxy oder Hydroxy steht,

$R^5$ für Wasserstoff, Alkyl, Halogen, Alkoxy oder Hydroxy steht und

$R^6$ für Wasserstoff, Alkyl, Halogen, Alkoxy oder Hydroxy steht.

10. Verfahren zur Herstellung von N-Pyrimidinyl-anilinen der Formel (II), dadurch gekennzeichnet, daß man Aniline der Formel

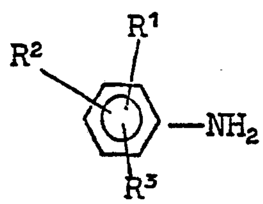

in welcher

$R^1$, $R^2$ und $R^3$ die oben angegebene Bedeutung haben,

mit Pyrimidin-2-yl-methyl-chloriden der Formel

Cl - $CH_2$ 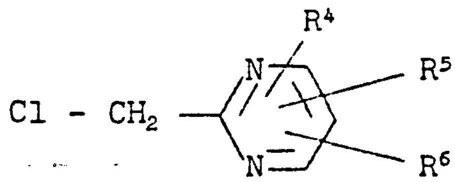 (V)

in welcher

$R^4$, $R^5$ und $R^6$ die oben angegebene Bedeutung haben,

in Gegenwart eines Säurebinders und gegebenenfalls in Gegenwart eines Verdünnungsmittels umsetzt.